# EUROPÄISCHE PATENTSCHRIFT

(11) **EP 1 266 882 B1**
(45) Veröffentlichungstag und Bekanntmachung des Hinweises auf die Patenterteilung: **06.04.2005**
(21) Anmeldenummer: 02012589.4
(22) Anmeldetag: 06.06.2002
(51) Int. Cl.: C07C 67/303

(54) **Verfahren zur Herstellung von Cyclohexandicarbonsäureestern**
Process for the preparation of cyclohexanedicarboxylic acid esters
Procédé de préparation d'esters de l'acide cyclohexanedicarboxylique

(30) Priorität: 16.06.2001 DE 10129129
(43) Veröffentlichungstag der Anmeldung: 18.12.2002
(73) Patentinhaber: Celanese Chemicals Europe GmbH, 60439 Frankfurt am Main (DE)
(72) Erfinder: Bohnen, Hans, Dr., 47441 Moers (DE); Klein, Thomas, 46149 Oberhausen (DE); Bergrath, Klaus, 46147 Oberhausen (DE)

(56) Entgegenhaltungen:
- US-A- 2 070 770

## Beschreibung

Die vorliegende Erfindung betrifft ein Verfahren zur Herstellung von Cyclohexandicarbonäureestem (Hexahydrophthalsäurediestem) durch Veresterung von Benzoldicarbonsäuren bzw. deren Anhydriden mit linearen oder verzweigten aliphatischen C₄- bis C₁₄- Monoalkoholen und anschließende Hydrierung. Die Umsetzung der aromatischen Dicarbonsäureester mit Wasserstoff erfolgt in Gegenwart ausgewählter Nickelkatalysatoren in der Gasoder in der Flüssigphase.

Hydrierte Benzoldicarbonsäureester finden unter anderem als Zwischenprodukte in der industriellen organischen Chemie und als Weichmacher für Kunststoffe, für Beschichtungsmittel, Dichtungsmassen und in Kautschukund Gummiartikeln Anwendung.

Die Wirksamkeit der Weichmacher beruht darauf, dass sie die physikalischen Eigenschaften der hochpolymeren thermoplastischen Stoffe ohne mit ihnen zu reagieren verändern, vorzugsweise durch ihr Löse- und Quellvermögen. Aus Thermoplast und Weichmacher bildet sich ein homogenes System aus, dessen thermoplastischer Bereich gegenüber dem ursprünglichen Polymeren zu niedrigeren Temperaturen verschoben ist mit dem Ergebnis, dass z.B. Formveränderungsvermögen und Elastizität des Polymerisats erhöht werden und seine Härte verringert wird.

Um Weichmachern weite Anwendungsmöglichkeiten zu eröffnen, müssen sie eine Reihe Anforderungen erfüllen. Im Idealfall sollten sie geruchlos, farblos, licht-, kälte- und wärmebeständig sein. Überdies erwartet man, dass sie sich unter der Einwirkung von Feuchtigkeit bzw. Wasser nicht verändern, schwer brennbar, wenig flüchtig und insbesondere auch toxikologisch unbedenklich sind. Weiterhin soll ihre Herstellung einfach sein und, um ökologischen Ansprüchen zu genügen, die Bildung lästiger Abfallstoffe, wie nicht verwertbare Nebenprodukte und Schadstoffe enthaltende Abwässer, vermeiden.

Zu den industriell in sehr großem Umfang als Weichmacher genutzten Verbindungen gehören die Ester der isomeren Phthalsäuren, insbesondere Ester die sich von der ortho-Phthalsäure und von Weichmacheralkoholen, d.h. linearen oder verzweigten primären Alkoholen mit 4 bis 14 Kohlenstoffatomen im Molekül ableiten. Man setzt sie als einheitliche Verbindungen oder als Gemische unterschiedlicher Ester ein. Ihrer unbegrenzten Verwendung steht jedoch entgegen, dass in jüngster Zeit Vorbehalte gegen die gesundheitliche Unbedenklichkeit dieser Stoffklasse geäußert wurden. Daher verbietet sich ihr Einsatz in Verbindung mit Lebensmitteln z.B. als Verpackungsmaterial und in anderen Erzeugnissen, deren Gebrauch aus Gründen der Gesundheitsvorsorge besonderer Sorgfalt unterliegt. Zu solchen Produkten gehören z.B. Artikel des täglichen Bedarfs, wie Haushaltsgegenstände und Gegenstände für die Versorgung und Betreuung von Kindern, darin eingeschlossen Spielzeug, sowie Erzeugnisse, die im medizinischen Bereich eingesetzt werden. Infolgedessen verwendet man als Weichmacher für Hilfs- und Fertigprodukte aus Thermoplasten die für diese speziellen Anwendungsgebiete bestimmt sind, nicht Phthalate sondern weicht auf toxisch unbedenkliche Verbindungen aus. Zu ihnen gehören die Ester der Citronensäure, deren Anwendung aus wirtschaftlichen Gründen auf Spezialgebiete, z.B. Kinderspielzeug, beschränkt ist. Breiteren Einsatz versprechen wegen ihrer weitaus größeren Verfügbarkeit die Ester der Cyclohexandi- und -polycarbonsäuren (Hexahydrobenzoldi- und -polycarbonsäuren).

Die Verwendung eines Cyclohexandicarbonsäureesters, nämlich des Cyclohexan-1,2-dicarbonsäuredi(2-ethylhexyl)esters als Weichmacher ist aus der DE-A 12 63 296 bekannt. Eine umfangreiche Zusammenstellung von Estern der Cyclohexandi-, -tri- und -tetracarbonsäuren, die als Weichmacher für Kunststoffe geeignet sind, findet sich in der DE 199 27 977 A1. In dieser Veröffentlichung wird insbesondere auf die toxikologisch günstigen Eigenschaften der genannten Verbindungen hingewiesen.

Die Herstellung von Cyclohexandicarbonsäureestern erfolgt üblicherweise durch katalytische Hydrierung von Estern der Benzolcarbonsäuren. Ein solches Verfahren ist z.B. Gegenstand der DE 28 23 165 A1. Als Katalysatoren werden bei dieser Arbeitsweise Nickel insbesondere aber die Platinmetalle Ruthenium, Rhodium oder Palladium eingesetzt, die auf einem Lithium-Aluminium-Spinell (LiAl₅O₆)-Träger aufgebracht sind.

Auch aus US-A-2,070,770 ist bekannt, Dialkylphthalate in Gegenwart von aktiven Nickelkatalysatoren zu den entsprechenden Hexahydrophthalaten zu hydrieren.

Ein weiteres Verfahren zur Herstellung von Cyclohexandicarbonsäureestern wird in der DE 197 56 913 A1 beschrieben. Nach dieser Veröffentlichung setzt man Benzoldicarbonsäureester als Einzelverbindung oder im Gemisch mit anderen Benzoldicarbonsäureestern mit einem Wasserstoff enthaltenden Gas in Gegenwart eines Katalysators um, der mindestens ein Metall der VIII. Nebengruppe des Periodensystems allein oder zusammen mit mindestens einem Metall der I. oder VII. Nebengruppe des Periodensystems, aufgebracht auf einem Träger, der Makroporen aufweist, umfasst. Als katalytisch aktives Metall wird insbesondere Ruthenium verwendet.

Die bekannten Prozesse erfordern den Einsatz reiner Benzoldicarbonsäureester. Daher müssen die Ausgangsverbindungen nach ihrer Synthese und vor der Umsetzung mit Wasserstoff aufwändigen Reinigungsschritten unterworfen werden. Überdies steht im Vordergrund der Verfahren des Standes der Technik die Verwendung von Platinmetallkatalysatoren, insbesondere Rutheniumkatalysatoren zur Durchführung der Hydrierung. Um die Edelmetallverluste über die Gesamtreaktion in engen Grenzen zu halten ist eine möglichst quantitative Wiedergewinnung der Katalysatoren unerlässlich. Die hierzu erforderlichen Maßnahmen bedingen die Ausübung weiterer Verfahrensschritte und, in Verbindung damit, die Bereitstellung zusätzlicher Anlagenteile. Unter diesen Umständen besteht ein Interesse daran, den Herstellungsprozess zu vereinfachen und durch geeignete Reaktionsführung die Anzahl der Reaktionsschritte auf ein Mindestmaß zu begrenzen und dadurch die Wirtschaftlichkeit des Verfahrens zu erhöhen.

Aufgabe der vorliegenden Erfindung ist es ein Verfahren zur Herstellung cycloaliphatscher Dicarbonsäureester aus den entsprechenden Benzoldicarbonsäuren bzw. deren Anhydriden bereitzustellen, das die Benzoldicarbonsäureestersynthese mit der sich daran anschließenden Hydrierung zu einem integralen Prozess in der Weise verknüpft, dass gesonderte Isolierungs- und Reinigungsschritte entbehrlich sind.

Erfindungsgemäß wird die vorstehend beschriebene Aufgabe gelöst durch ein Verfahren zur Herstellung von Cyclohexandicarbonsäureestern durch Veresterung einer Benzoldicarbonsäure oder eines Benzoldicarbonsäureanhydrids mit einem aliphatischen C₄- bis C₁₄-Monoalkohol in Gegenwart einer Säure oder eines Titan, Zirconium oder Zinn enthaltenden Katalysators bei Temperaturen von 100 bis 250°C unter Entfernung gegebenenfalls gebildeten Wassers und Neutralisation des Reaktionsgemisches nach Beendigung der Veresterung durch Zusatz eines alkalischen Reagenzes. Die neue Arbeitsweise ist dadurch gekennzeichnet, dass man das neutralisierte Veresterungsgemisch gegebenenfalls nach Entfernung von der Neutralisation herrührenden Wassers ohne weitere Vorbehandlung in Gegenwart eines Nickelkatalysators hydriert und anschließend den Cyclohexandicarbonsäureester durch Abtrennung der Nebenprodukte aus dem Reaktionsgemisch isoliert.

Das neue Verfahren ermöglicht es, ausgehend von Benzoldicarbonsäuren oder deren Anhydriden, in aufeinanderfolgenden Reaktionsschritten Ester der Cyclohexandicarbonsäuren herzustellen. Es verdient besonders hervorgehoben zu werden, dass eine Isolierung von Zwischenverbindungen nicht erforderlich ist, weder, aus verfahrenstechnischen Gründen, noch im Zusammenhang mit intermediären Reinigungsschritten. Dennoch werden als Reaktionsprodukte hochreine Ester cycloaliphatischer Dicarbonsäuren erhalten, die alle Voraussetzungen erfüllen, um sie erfolgreich als Weichmacher einzusetzen. Die Ester zeichnen sich insbesondere durch hervorragende Farbeigenschaften wie Farbstabilität und durch äußerst geringe Leitfähigkeit aus, Eigenschaften, die ihnen eine breite Anwendung in der Kunststoffverarbeitung erschließen.

Das neue Verfahren geht von Dicarbonsäuren oder Dicarbonsäureanhydriden des Benzols und aliphatischen Monoalkoholen mit 4 bis 14 Kohlenstoffatomen im Molekül als Einsatzverbindungen aus. Unter dem Begriff Dicarbonsäuren des Benzols werden die stellungsisomeren Verbindungen Phthalsäure (o-Phthalsäure), Isophthalsäure (m-Phthalsäure) und Terephthalsäure (p-Phthalsäure), insbesondere die o-Phthalsäure verstanden. Als Alkoholkomponente der Ester kommen sowohl lineare als auch verzweigte Verbindungen der genannten Molekülgröße in Betracht. Bevorzugt werden Alkohole mit 4 bis 10 Kohlenstoffatomen im Molekül, z.B. n-Butanol, n-Octanol-(1), n-Octanol-(2), 2-Ethylhexanol, n- und iso- Nonylalkohole, n-und iso- Decylalkohole und die sogenannten Oxoalkohole, d.h. Gemische linearer und verzweigter Alkohole entsprechender Molekülgröße, die durch Oxoreaktion aus Olefinen und anschließende Hydrierung erhalten werden.

Die Herstellung der Ester erfolgt in bekannter Weise durch Umsetzung von Dicarbonsäure oder Dicarbonsäureanhydrid mit Alkohol, der häufig im Überschuss vorliegt, in Gegenwart eines Katalysators. Nach den klassischen Verfahren sind die Katalysatoren Säuren, z.B. Schwefelsäure. Moderne Verfahren setzen metallhaltige Veresterungskatalysatoren ein, insbesondere Zinn, Titan und Zirconium in Form der feinverteilten Metalle, als Salze, Oxide oder im Reaktionsgemisch lösliche organische Verbindungen.

Die Reaktion der in Mischung vorliegenden Ausgangsstoffe erfolgt gewöhnlich bei 100 bis 250°C, unter Rühren, in einer oder in mehreren, durch unterschiedliche Temperaturbereiche charakterisierten Stufen. Entstehendes Reaktionswasser wird zweckmäßig als Azeotrop entfernt, z.B. mit Cyclohexan oder dem zur Veresterung eingesetzten Alkohol als Azeotropkomponenten.

Nach Beendigung der Reaktion enthält das Veresterungsgemisch neben dem erwünschten Reaktionsprodukt, dem Diester, insbesondere noch überschüssigen Alkohol und den Katalysator.

Zur Neutralisation der sauren Bestandteile und zur Entfernung des Katalysators setzt man das Reaktionsgemisch mit einem alkalischen Reagenz um. Geeignete alkalische Verbindungen sind Alkali- oder Erdalkalihydroxide oder Alkalicarbonate, die als wässrige Lösung zum Einsatz gelangen. Die Konzentration der alkalischen Verbindungen in der Lösung beträgt gewöhnlich 5 bis 20 Gew.-% bezogen auf die Lösung. Die Menge des zuzusetzenden Neutralisationsmittels richtet sich nach dem Anteil saurer Komponenten im Rohprodukt. Dieser Anteil wird in Form der Säurezahl (nach DIN 53169) bestimmt. Gemäß dem neuen Verfahren ist es nicht notwendig, das alkalische Reagenz dem Veresterungsgemisch in exakt der Menge zuzusetzen, die den vorhandenen H⁺-Ionen zur Neutralisation äquivalent ist. Vielmehr kann es im Überschuss verwendet werden, der bis zur zehnfachen und nach einer bewährten Ausführungsform der neuen Arbeitsweise der zwei- bis vierfachen Menge entspricht, die stöchiometrisch erforderlich ist, um die H⁺-lonen zu neutralisieren.

Im Anschluss an die Neutralisation wird das rohe Veresterungsgemisch ohne weitere Vorbehandlung mit Wasserstoff umgesetzt. Es empfiehlt sich lediglich von der Neutralisation herrührendes Wasser, sofern es als eigene Phase vorliegt, zuvor zu entfernen, zweckmäßig durch einfache Trennung von organischer und wässriger Phase.

Erfindungsgemäß führt man die Hydrierung in Gegenwart von Nickelkatalysatoren durch. Das katalytisch aktive Metall ist auf einem Träger aufgebracht, im allgemeinen in einer Menge von etwa 5 bis etwa 70 Gew.-%, vorzugsweise etwa 10 bis etwa 65 Gew.-% und insbesondere etwa 20 bis etwa 60 Gew.-% jeweils bezogen auf das Gesamtgewicht des Katalysators. Als Katalysatorträger eignen sich alle herkömmlichen Trägermaterialien, z.B. Aluminiumoxid, Aluminiumoxidhydrate in ihren verschiedenen Erscheinungsformen, Siliciumdioxid, Polykieselsäuren (Kieselgele) einschließlich Kieselgur, Kieselxerogele, Magnesiumoxid, Zinkoxid, Zirconiumoxid und Aktivkohle. Neben den Hauptkomponenten Nickel und Trägermaterial können die Katalysatoren noch Zusatzstoffe in untergeordneten Mengen enthalten, die z.B. der Verbesserung ihrer Hydrieraktivität und/oder ihrer Standzeit und/oder ihrer Selektivität dienen. Derartige Zusatzstoffe sind bekannt, zu ihnen gehören z.B. die Oxide des Natriums, Kaliums, Magnesiums, Calciums, Bariums, Zinks, Aluminiums, Zirconiums und Chroms. Sie werden dem Katalysator im allgemeinen in einem Anteil von insgesamt 0,1 bis 50 Gew.-Teile bezogen auf 100 Gew.-Teile Nickel zugesetzt.

Die Herstellung der im Verfahren der Erfindung eingesetzten Katalysatoren erfolgt nach konventionellen Prozessen. So können der hydrieraktive Bestandteil Nickel und gegebenenfalls vorhandene Zusatzstoffe zusammen mit dem Träger aus wässrigen Lösungen, die die Katalysatorbestandteile in entsprechender Zusammensetzung als Salze gelöst enthalten, ausgefällt werden. Eine andere mögliche Arbeitsweise geht von suspendiertem Trägermaterial aus, auf das die hydrieraktive Komponente wahlweise zusammen mit Zusatzstoffen gefällt wird. Schließlich kann man geeignete Katalysatoren auch durch Tränken von Trägerstoffen mit Lösungen des hydrieraktiven Metalls und möglicher Zusatzstoffe gewinnen. Die Katalysatoren gelangen üblicherweise geformt z.B. als Tabletten oder Stränge oder gekörnt zum Einsatz.

Erfindungsgemäß wird die Hydrierung bei Temperaturen von etwa 50 bis etwa 250°C, vorzugsweise 80 bis 220°C durchgeführt. Der angewandte Druck beträgt in der Regel 1 MPa und mehr, vorzugsweise etwa 2 bis 30 MPa.

Das neue Verfahren wird diskontinuierlich oder kontinuierlich in flüssiger Phase mit suspendierten Katalysatoren oder in flüssiger oder gasförmiger Phase mit fest angeordneten Katalysatoren durchgeführt; die kontinuierliche Arbeitsweise wird bevorzugt.

Bei diskontinuierlicher Verfahrensführung verwendet man, bezogen auf den Ausgangsester bzw. das Estergemisch, 1 bis 10, vorzugsweise 2 bis 6 Gew.-% Nickel in Form der vorstehend beschriebenen Katalysatoren. Bei kontinuierlicher Arbeitsweise setzt man je Liter Katalysator und Stunde etwa 0,05 bis etwa 5,0 kg des Ausgangsesters bzw. Estergemisches ein, bevorzugt werden etwa 0,1 bis 2,0 kg Ester je Liter Katalysator und Stunde.

Die Hydrierung erfolgt vorzugsweise mit reinem Wasserstoff. Es können jedoch auch Gemische verwendet werden, die freien Wasserstoff und daneben unter den Hydrierbedingungen inerte Bestandteile enthalten. In jedem Fall ist dafür Sorge zu tragen, dass das Hydriergas frei von Katalysatorgiften wie Schwefelverbindungen oder Kohlenmonoxid in schädlichen Mengen ist.

Das Ausgangsmaterial Ester bzw. Estergemisch kann als solches oder zusammen mit einem Lösungs- oder Verdünnungsmittel eingesetzt werden, wobei die zuletzt genannte Variante bevorzugt wird. Die Auswahl der Lösungs- oder Verdünnungsmittel, die reine Substanzen aber auch Substanzgemische sein können, ist nicht kritisch sofern sichergestellt ist, dass sie mit dem Einsatzstoff eine homogene Lösung bilden. Beispiele für geeignete Lösungs- oder Verdünnungsmittel sind aliphatische Alkohole mit bis zu 10 Kohlenstoffatomen im Molekül, insbesondere C₃- bis C₆-Alkohole, z.B. die isomeren Propanole und Butanole sowie n-Hexanol und lineare oder cyclische Ether wie Tetrahydrofuran oder Dioxan. Die Menge des eingesetzten Lösungs- oder Verdünnungsmittels kann entsprechend den apparativen und verfahrenstechnischen Gegebenheiten frei gewählt werden, im allgemeinen setzt man Lösungen ein, die 10 bis 75 Gew.-% Einsatzester oder Einsatzestergemisch enthalten. Besonders bewährt hat es sich im Rahmen des erfindungsgemäßen Verfahrens das bei der Hydrierung entstandene Produkt als Lösungs- oder Verdünnungsmittel zu verwenden. In diesem Fall wird, bezogen auf das Gewicht der zu hydrierenden Verbindung, zweckmäßig die 1- bis 30-fache, vorzugsweise die 5- bis 20-fache und insbesondere die 5-bis 10-fache Menge des hydrierten Produktes als Lösungs- und Verdünnungsmittel zugesetzt.

Zur Gewinnung des reinen Cyclohexandicarbonsäureesters destilliert man aus dem Hydrierungsgemisch die leichter als der Cyclohexandicarbonsäureester siedenden Bestandteile ab. Diese Destillation kann sowohl unter Normaldruck als auch unter vermindertem Druck erfolgen. Im allgemeinen genügt ein Destillationsschritt, lediglich in Ausnahmefällen kann eine zweite Destillationsstufe erforderlich werden.

Im Folgenden wird das erfindungsgemäße Verfahren anhand einiger Beispiele näher erläutert, es ist jedoch nicht auf die beschriebenen Ausführungsformen beschränkt.

### BEISPIELE

Die in den folgenden Beispielen als Ausgangsmaterial verwendeten Veresterungsgemische waren durch Umsetzung von Phthalsäureanhydrid mit 2-Ethylhexanol in Gegenwart von Schwefelsäure als Katalysator und anschließende Neutralisation des Reaktionsproduktes hergestellt worden.

Das in die Hydrierung eingesetzte Veresterungsgemisch hatte folgende, gaschromatographisch ermittelte Zusammensetzung (Angaben in Gew.-%); DEHP steht für Di(2-ethylhexyl)phthalat.

| | |
|---|---|
| Vorlauf | 0,89 |
| 2-Ethylhexanol | 11,34 |
| Zwischenlauf | 0,37 |
| DEHP | 87,40 |

Die Umsetzung mit Wasserstoff wurde in flüssiger Phase bei Temperaturen von 140°C und 8 MPa Druck durchgeführt Die Hydrierzeit richtete sich nach der Wasserstoffaufnahme, war sie beendet, wurde zur Vervollständigung der Reaktion noch 30 min nachhydriert, anschließend abgekühlt, entspannt und das vom Katalysator abfiltrierte Hydriergemisch gaschromatographisch analysiert.

### BEISPIELE 1 bis 4

In diesen Beispielen wurden wechselnde Mengen eines 55 Gew.-% Nickel, 6 Gew.-% Magnesiumoxid, ca. 34 Gew.-% Kieselgur enthaltenden Katalysators eingesetzt.

### Beispiel 1

2 Gew.-% Katalysator, bezogen auf eingesetztes Veresterungsgemisch

| | |
|---|---|
| Vorlauf | 3,86 |
| 2-Ethylhexanol | 10,90 |
| Zwischenlauf | 0,68 |
| DEHP | 77,11 |
| DEHP hydriert | 7,45 |

### Beispiel 2

3 Gew.-% Katalysator, bezogen auf eingesetztes Veresterungsgemisch

| | |
|---|---|
| Vorlauf | 0,59 |
| 2-Ethylhexanol | 10,90 |
| Zwischenlauf | 0,74 |
| DEHP | 42,32 |
| DEHP hydriert | 45,45 |

### Beispiel 3

4 Gew.-% Katalysator, bezogen auf eingesetztes Veresterungsgemisch

| | |
|---|---|
| Vorlauf | 0,72 |
| 2-Ethylhexanol | 11,99 |
| Zwischenlauf | 0,41 |
| DEHP | 0,42 |
| DEHP hydriert | 86,46 |

### Beispiel 4

5 Gew.-% Katalysator, bezogen auf eingesetztes Veresterungsgemisch

| | |
|---|---|
| Vorlauf | 1,40 |
| 2-Ethylhexanol | 10,46 |
| Zwischenlauf | 0,52 |
| DEHP | 0,16 |
| DEHP hydriert | 87,46 |

### BEISPIELE 5 bis 8

In diesen Beispielen wurden wechselnde Mengen eines 60 Gew.-% Nickel, etwa 27 Gew.-% Kieselgur und etwa 3 Gew.-% Aluminiumoxid enthaltenden Katalysators eingesetzt.

### Beispiel 5

2 Gew.-% Katalysator, bezogen auf eingesetztes Veresterungsgemisch

| | |
|---|---|
| Vorlauf | 0,74 |
| 2-Ethylhexanol | 11,98 |
| Zwischenlauf | 0,69 |
| DEHP | 48,78 |
| DEHP hydriert | 37,81 |

### Beispiel 6

3 Gew.-% Katalysator, bezogen auf eingesetztes Veresterungsgemisch

| | |
|---|---|
| Vorlauf | 0,36 |
| 2-Ethylexanol | 10,02 |
| Zwischenlauf | 0,44 |
| DEHP | 0,39 |
| DEHP hydriert | 88,79 |

### Beispiel 7

4 Gew.-% Katalysator, bezogen auf eingesetztes Veresterungsgemisch

| | |
|---|---|
| Vorlauf | 0,73 |
| 2-Ethylhexanol | 11,47 |
| Zwischenlauf | 0,53 |
| DEHP | 0,43 |
| DEHP hydriert | 86,84 |

### Beispiel 8

5 Gew.-% Katalysator, bezogen auf eingesetztes Veresterungsgemisch

| | |
|---|---|
| Vorlauf | 0,10 |
| 2-Ethylhexanol | 7,76 |
| Zwischenlauf | 0,46 |
| DEHP | 0,12 |
| DEHP hydriert | 91,56 |

Durch Abdestillieren der leichter als das gewünschte Reaktionsprodukt siedenden Bestandteile des Hydrierungsgemisches bei 120°C und 0,01 MPa wurde der Di(2-ethylhexyl)ester der Cyclohexan-1,2-dicarbonsäure in Weichmacherqualität isoliert.

## Patentansprüche

1. Verfahren zur Herstellung von Cyclohexandicarbonsäureestern durch Veresterung einer Benzoldicarbonsäure oder eines Benzoldicarbonsäureanhydrids mit einem aliphatischen C₄- bis C₁₄-Monoalkohol in Gegenwart einer Säure oder eines Titan, Zirconium oder Zinn enthaltenden Katalysators bei Temperaturen von 100 bis 250°C unter Entfernung gegebenenfalls gebildeten Wassers und Neutralisation des Reaktionsgemisches nach Beendigung der Veresterung durch Zusatz eines alkalischen Reagenzes **dadurch gekennzeichnet, dass** man das neutralisierte Veresterungsgemisch gegebenenfalls nach Entfernung von der Neutralisation herrührenden Wassers ohne weitere Vorbehandlung in Gegenwart eines Nickelkatalysators hydriert und anschließend den Cyclohexandicarbonsäureester durch Abtrennung der Nebenprodukte aus dem Reaktionsgemisch isoliert.

2. Verfahren nach Anspruch 1 **dadurch gekennzeichnet, dass** die Benzoldicarbonsäure bzw. das Benzoldicarbonsäureanhydrid o-Phthalsäure bzw. o-Phthalsäureanhydrid ist.

3. Verfahren nach Anspruch 1 oder 2 **dadurch gekennzeichnet, dass** der aliphatische Alkohol ein C₄- bis C₁₀- Alkohol ist.

4. Verfahren nach mindestens einem der Ansprüche 1 bis 3 **dadurch gekennzeichnet, dass** dem Veresterungsgemisch das alkalische Reagenz in der bis zur zehnfachen Menge zugesetzt wird, die stöchiometrisch erforderlich ist, um die vorhandenen H⁺- lonen zu neutralisieren.

5. Verfahren gemäß Anspruch 4 **dadurch gekennzeichnet, dass** dem Veresterungsgemisch das alkalische Reagenz in der zwei- bis vierfachen Menge zugesetzt wird, die stöchiometrisch erforderlich ist, um die vorhandenen H⁺-Ionen zu neutralisieren.

6. Verfahren nach mindestens einem der Ansprüche 1 bis 5 **dadurch gekennzeichnet, dass** der Katalysator 5 bis 70 Gew.-% Nickel, bezogen auf das Gesamtgewicht des Katalysators, enthält.

7. Verfahren nach Anspruch 6 **dadurch gekennzeichnet, dass** der Katalysator 10 bis 65 Gew.-% Nickel, bezogen auf das Gesamtgewicht des Katalystors, enthält.

8. Verfahren nach Anspruch 7 **dadurch gekennzeichnet, dass** der Katalysator 20 bis 60 Gew.-% Nickel, bezogen auf das Gesamtgewicht des Katalysators, enthält.

9. Verfahren nach mindestens einem der Ansprüche 1 bis 8 **dadurch gekennzeichnet, dass** die Hydrierung bei Temperaturen von 50 bis 250°C und bei Drücken von mindestens 1 MPa durchgeführt wird.

10. Verfahren nach Anspruch 9 **dadurch gekennzeichnet, dass** die Hydrierung bei Temperaturen von 80 bis 220°C und bei Drücken von 2 bis 30 MPa durchgeführt wird.

11. Verfahren nach mindestens einem der Ansprüche 1 bis 10 **dadurch gekennzeichnet, dass** die Hydrierung kontinuierlich in flüssiger oder gasförmiger Phase erfolgt.

12. Verfahren nach Anspruch 11 **dadurch gekennzeichnet, dass** bei diskontinuierlicher Arbeitsweise bezogen auf eingesetzten Ester bzw. eingesetztes Estergemisch 1 bis 10 Gew.-% Nickel in Form von Nickelkatalysator verwendet werden.

13. Verfahren nach Anspruch 12 **dadurch gekennzeichnet, dass**, bezogen auf eingesetzten Ester bzw. eingesetztes Estergemisch, 2 bis 6 Gew.-% Nickel in Form von Nickelkatalysator verwendet werden.

14. Verfahren nach Anspruch 11 **dadurch gekennzeichnet, dass** bei kontinuierlicher Arbeitsweise je Liter Katalysator und Stunde 0,05 bis 5,0 kg Ester bzw. Estergemisch eingesetzt werden.

15. Verfahren nach Anspruch 14 **dadurch gekennzeichnet, dass** je Liter Katalysator und Stunde 0,1 bis 2,0 kg Ester bzw. Estergemisch eingesetzt werden.

16. Verfahren nach mindestens einem der Ansprüche 1 bis 15 **dadurch gekennzeichnet, dass** der Ester bzw. das Estergemisch in einem Lösungs- oder Verdünnungsmittel gelöst in der Hydrierung eingesetzt wird.

17. Verfahren nach Anspruch 16 **dadurch gekennzeichnet, dass** die Lösung 10 bis 75 Gew.-% Ester bzw. Estergemisch enthält.

18. Verfahren nach Anspruch 16 **dadurch gekennzeichnet, dass** das Lösungs- oder Verdünnungsmittel das bei der Hydrierung des Esters bzw. Estergemischs entstandene Produkt ist.

19. Verfahren nach Anspruch 18 **dadurch gekennzeichnet, dass** bezogen auf das Gewicht des zu hydrierenden Esters bzw. Estergemisches die 1- bis 30-fache Menge des hydrierten Produktes als Lösungs- bzw. als Verdünnungsmittel eingesetzt wird.

20. Verfahren nach Anspruch 19 **dadurch gekennzeichnet, dass**, bezogen auf das Gewicht des zu hydrierenden Esters bzw. Estergemisches die 5- bis 20-fache Menge des hydrierten Produktes als Lösungs- bzw. als Verdünnungsmittel eingesetzt wird.

21. Verfahren nach Anspruch 20 **dadurch gekennzeichnet, dass**, bezogen auf das Gewicht des zu hydrierenden Esters bzw. Estergemisches die 5- bis 10-fache Menge des hydrierten Produktes als Lösungs- bzw. als Verdünnungsmittel eingesetzt wird.

## Claims

1. A process for preparing cyclohexanedicarboxylic esters by esteryfing a benzenedicarboxylic acid or a benzenedicarboxylic anhydride with an aliphatic C₄-C₁₄ monoalcohol in the presence of an acid or of a catalyst containing titanium, zirconium or tin, at temperatures of from 100 to 250°C, with removal of any water formed and neutralization of the reaction mixture once the esterification has been completed by adding an alkaline reagent, which comprises hydrogenating the neutralized esterification mixture where appropriate after removal of the water generated by the neutralization without other pretreatment in the presence of a nickel catalyst, and then isolating the cyclohexanedicarboxylic ester by separating off the by-products from the reaction mixture.

2. The process as claimed in claim 1, wherein the benzenedicarboxylic acid or the benzenedicarboxylic anhydride is o-phthalic acid or o-phthalic anhydride.

3. The process as claimed in claim 1 or 2, wherein the aliphatic alcohol is a C₄-C₁₀ alcohol.

4. The process as claimed in at least one of claims 1 to 3, wherein the amount of the alkaline reagent added to the esterification mixture is up to ten times the amount stoichiometrically required to neutralize the H⁺ ions present.

5. The process according to claim 4, wherein the amount of the alkaline reagent added to the esterification mixture is from two to four times the amount stoichiometrically required to neutralize the H⁺ ions present.

6. The process as claimed in at least one of claims 1 to 5, wherein the catalyst comprises, based on the total weight of the catalyst, from 5 to 70% by weight of nickel.

7. The process as claimed in claim 6, wherein the catalyst comprises, based on the total weight of the catalyst, from 10 to 65% by weight of nickel.

8. The process as claimed in claim 7, wherein the catalyst comprises, based on the total weight of the catalyst, from 20 to 60% by weight of nickel.

9. The process as claimed in at least one of claims 1 to 8, wherein the hydrogenation is carried out at temperatures of from 50 to 250°C and at pressures of at least 1 MPa.

10. The process as claimed in claim 9, wherein the hydrogenation is carried out at temperatures of from 80 to 220°C and at pressures of from 2 to 30 MPa.

11. The process as claimed in at least one of claims 1 to 10, wherein the hydrogenation takes place continuously in the liquid or gas phase.

12. The process as claimed in claim 11, wherein use is made, based on ester or ester mixture used, of from 1 to 10% by weight of nickel in the form of nickel catalyst, in the case of a batchwise procedure.

13. The process as claimed in claim 12, wherein use is made, based on ester or ester mixtures used, of from 2 to 6% by weight of nickel in the form of nickel catalyst.

14. The process as claimed in claim 11, wherein, per liter of catalyst and hour, use is made of from 0.05 to 5.0 kg of ester or ester mixture, in the case of a continuous procedure.

15. The process as claimed in claim 14 wherein, per liter of catalyst and hour, use is made of from 0.1 to 2.0 kg of ester or ester mixture.

16. The process as claimed in at least one or claims 1 to 15, wherein the ester or ester mixture used in the hydrogenation has been dissolved in a solvent or diluent.

17. The process as claimed in claim 16, wherein the solution comprises from 10 to 75% by weight of ester or ester mixture.

18. The process as claimed in claim 16, wherein the solvent or diluent is the product produced during hydrogenation of the ester or of the ester mixture.

19. The process as claimed in claim 18, wherein the amount of the hydrogenated product used as solvent or as diluent is from 1 to 30 times, the weight of the ester or ester mixture to be hydrogenated.

20. The process as claimed in claim 19, wherein the amount of the hydrogenated product used as solvent or as diluent is from 5 to 20 times, the weight of the ester or ester mixture to be hydrogenated.

21. The process as claimed in claim 20, wherein the amount of the hydrogenated product used as solvent or as diluent is from 5 to 10 times, the weight of the ester or ester mixture to be hydrogenated.

## Revendications

1. Procédé pour la préparation d'esters d'acides cyclohexanedicarboxyliques par estérification d'un acide benzènedicarboxylique ou d'un anhydride d'acide benzènedicarboxylique avec un monoalcool aliphatique en C₄ à C₁₄, en présence d'un acide ou d'un catalyseur contenant du titane, du zirconium ou de l'étain à des températures de 100 à 250°C avec élimination d'eau éventuellement formée et neutralisation du mélange réactionnel à la fin de l'estérification par addition d'un réactif alcalin, **caractérisé en ce qu'**on hydrogène le mélange d'estérification neutralisé le cas échéant après élimination de l'eau provenant de la neutralisation, sans autre prétraitement, en présence d'un catalyseur de nickel et on isole ensuite l'ester d'acide cyclohexanedicarboxylique par séparation des produits secondaires du mélange réactionnel.

2. Procédé selon la revendication 1, **caractérisé en ce que** l'acide benzènedicarboxylique ou l'anhydride de l'acide benzènedicarboxylique est l'acide o-phtalique ou l'anhydride de l'acide o-phtalique.

3. Procédé selon la revendication 1 ou 2, **caractérisé en ce que** l'alcool aliphatique est un alcool en C₄ à C₁₀.

4. Procédé selon au moins l'une quelconque des revendications 1 à 3, **caractérisé en ce que** le réactif alcalin est ajouté au mélange d'estérification en une quantité allant jusqu'à dix fois la quantité stoechiométriquement nécessaire pour neutraliser les ions H⁺ présents.

5. Procédé selon la revendication 4, **caractérisé en ce que** le réactif alcalin est ajouté au mélange d'estérification en une quantité représentant deux à quatre fois la quantité stoechiométriquement nécessaire pour neutraliser les ions H⁺ présents.

6. Procédé selon au moins l'une quelconque des revendications 1 à 5, **caractérisé en ce que** le catalyseur contient 5 à 70% en poids de nickel par rapport au poids total du catalyseur.

7. Procédé selon la revendication 6, **caractérisé en ce que** le catalyseur contient 10 à 65% en poids de nickel par rapport au poids total du catalyseur.

8. Procédé selon la revendication 7, **caractérisé en ce que** le catalyseur contient 20 à 60% en poids de nickel, par rapport au poids total du catalyseur.

9. Procédé selon au moins l'une quelconque des revendications 1 à 8, **caractérisé en ce que** l'hydrogénation est réalisée à des températures de 50 à 250°C et des pressions d'au moins 1 MPa.

10. Procédé selon la revendication 9, **caractérisé en ce que** l'hydrogénation est réalisée à des températures de 80 à 220°C et à des pressions de 2 à 30 MPa.

11. Procédé selon au moins l'une quelconque des revendications 1 à 10, **caractérisé en ce que** l'hydrogénation est réalisée en continu en phase liquide ou gazeuse.

12. Procédé selon la revendication 11, **caractérisé en ce que** lors du mode opératoire discontinu, on utilise, par rapport à l'ester ou au mélange d'esters utilisé 1 à 10% en poids de nickel sous forme d'un catalyseur au nickel.

13. Procédé selon la revendication 12, **caractérisé en ce qu'**on utilise, par rapport à l'ester ou au mélange d'ester utilisé, 2 à 6% en poids de nickel sous forme d'un catalyseur au nickel.

14. Procédé selon la revendication 11, **caractérisé en ce qu'**on utilise lors d'un mode opératoire en continu, par litre de catalyseur et par heure, 0,05 à 5,0 kg d'ester ou de mélange d'esters.

15. Procédé selon la revendication 14, **caractérisé en ce qu'**on utilise par litre de catalyseur et par heure, 0,1 à 2,0 kg d'ester ou de mélange d'esters.

16. Procédé selon au moins l'une quelconque des revendications 1 à 15, **caractérisé en ce que** l'ester ou le mélange d'esters est utilisé dans l'hydrogénation sous forme dissoute dans un solvant ou un diluant.

17. Procédé selon la revendication 16, **caractérisé en ce que** la solution contient 10 à 75% en poids d'ester ou de mélange d'esters.

18. Procédé selon la revendication 16, **caractérisé en ce que** le solvant ou le diluant est le produit formé lors de l'hydrogénation de l'ester ou du mélange d'esters.

19. Procédé selon la revendication 18, **caractérisé en ce que**, par rapport au poids de l'ester ou du mélange d'esters à hydrogéner, on utilise 1 à 30 fois la quantité du produit hydrogéné comme solvant ou diluant.

20. Procédé selon la revendication 19, **caractérisé en ce que**, par rapport au poids de l'ester ou du mélange d'esters à hydrogéner, on utilise 5 à 20 fois la quantité du produit hydrogéné comme solvant ou comme diluant.

21. Procédé selon la revendication 20, **caractérisé en ce que**, par rapport au poids de l'ester ou du mélange d'esters à hydrogéner, on utilise 5 à 10 fois la quantité du produit hydrogéné comme solvant ou comme diluant.
